# EUROPEAN PATENT SPECIFICATION

(11) **EP 3 746 139 B1**
(45) Date of publication and mention of the grant of the patent: **05.07.2023**
(21) Application number: 19703285.7
(22) Date of filing: 01.02.2019
(51) Int. Cl.: A61L 27/16, A61L 27/18, A61L 27/44, A61L 27/46, A61L 27/56

(54) **FUNCTIONALLY GRADED POLYMER KNEE IMPLANT FOR ENHANCED FIXATION, WEAR RESISTANCE, AND MECHANICAL PROPERTIES AND THE FABRICATION THEREOF**
FUNKTIONELL GESTUFTES POLYMERKNIEIMPLANTAT FÜR VERBESSERTE FIXIERUNG, VERSCHLEISSFESTIGKEIT UND MECHANISCHE EIGENSCHAFTEN UND DESSEN HERSTELLUNG
IMPLANT DE GENOU POLYMÈRE À GRADIENT FONCTIONNEL POUR DES PROPRIÉTÉS DE FIXATION, RÉSISTANCE À L'USURE, ET MÉCANIQUES AMÉLIORÉE ET SA FABRICATION

(30) Priority: 02.02.2018 US 201862625759 P
(43) Date of publication of application: 09.12.2020
(73) Proprietor: Evonik Operations GmbH, 45128 Essen (DE)
(72) Inventor: PRABHU, Balaji, Hoover, Alabama 35226 (US); WOOD, Andrew, Birmingham, Alabama 35211 (US); DADSETAN, Mahrokh, Birmingham, Alabama 35211 (US); KARAU, Andreas, 63571 Gelnhausen (DE); BANDI, Suneel, Lafayette, Indiana 47906 (US); ROSS, Kenneth, Miamisburg, Ohio 45342 (US); KNEBEL, Marc, 68542 Heddesheim (DE)
(74) Representative: Evonik Patent Association
(86) International application number: PCT/EP2019/052505
(87) International publication number: WO 2019/149890

(56) References cited:
- US-A- 5 176 710
- US-A1- 2007 255 412

## Description

The present disclosure relates to a device for use in orthopaedic implant applications that aims to provide an implant with higher wear-resistance, enhanced bone/implant interface osseo integration, a reduced likelihood of third-body wear, rapid post-operative healing, and more efficient load transfer from the implant to native bone structure in an effort to reduce the detrimental effects of stress-shielding on bone mineral density. The present invention is directed to increasing the wear-resistance of a knee implant component as well as initiating a physiological bond at the interface between the knee implant and the native ends of the distal femoral and/or tibial bone.

With an increasing aging demographic the need for artificial implants has seen rapid growth. A large portion of focus in this area has looked to develop or modify new or existing technologies, respectively, to supply improved materials and methods for large joint replacement / remediation. The large joints include those belonging to shoulder, hip, knee, and ankle with unique systems having been designed for each in an effort to provide beneficial devices to replace damaged, diseased, or dysfunctional native structures.

While metallic and/or ceramic based implants offer elevated mechanical properties necessary for large joint implants, they also carry with them deficiencies that decrease their effectiveness, service life, as well as introduce possible adverse biological reactions during activities carried out in a patient's everyday life (e.g., walking, squatting, etc.). For example, in total knee replacement, CoCr femoral knee implants (as well as those of other metallic and/or ceramic materials), are commonly used as they are easy to polish to a smooth surface, offer adequate fixation to the native bone, and provide reasonable wear-resistance.

Although these metallic and/or ceramic femoral knee implants dominate much of the total knee replacement market (as well as holding the vast majority of market in the other large joint reconstruction sectors), the mechanical properties that they possess introduce one way in which these materials can be detrimental to the patient's well-being in long-term applications. While the mechanical properties and wear-resistance of these types of materials are desirable, the degree to which their mechanical properties surpass that of native bone is commonly a cause for concern.

Specifically, the elevated mechanical properties of metallic and/or ceramic based femoral knee implants have been shown to consistently lead to a loss of bone mineral density in the distal femur. In accordance to Wolff's law, if the native bone is not under a load, it will undergo remodeling and regenerate a modified structure based on the newly applied load. In the case of metallic and/or ceramic based femoral knee implants, the implant carries the load typically applied to the distal end of the femoral bone therefore the distal end of the femoral bone remodels in accordance leading to reduced bone mineral density. The subsequent concerns following this loss of bone mineral density include the potential of implant loosening, onset of third-body wear, increased revisionary procedure difficulty, and/or implant failure and must be addressed.

Further, these metallic and/or ceramic based implants are problematic in that they are radiopaque, have the potential to evoke allergic reactions in patients, may release toxic sub-species into the patient's body, are prone to mechanically induced oxidation and corrosion, and have been recorded to fail mechanically due to many of these reasons as well as their proclivity to migrate away from the initial fixation site due to a lack of long-term fixation to the distal end of the femoral bone. The multitude of these factors has caused the current landscape to shift towards implementation of polymeric based materials in these particular applications. This shift in interest of implant materials is attributable to the mechanical properties of many polymeric based materials being able to be adjusted to closely mimic the native bone mechanical properties as well as their ease of processing, radiotransparency, lack of allergic reaction among patients, and removal of the oxidation and corrosion potential of currently used metallic based implants. Further, particular species of polymeric materials are bioinert therefore remove the potential for any adverse system effects upon implantation.

Of particular interest, the development of the thermoplastic polymeric species polyaryletherketones (PAEKs) and its derivatives (i.e., polyetheretherketone, polyetherketoneketone, polyetherketoneetheretherketone, and other derivative polymers) has seen recent increase. US5176710 A discloses an orthopaedic surgical implant device for replacing the articulating surface of a joint that fosters normal bone growth in the proximal tibia so the bone can support the device. The implant device can be made of PEEK, UHMWPE. The bone ingrowth is promoted by a porous thermoplastic coating. The PEEK material may be impregnated with glass and carbon fibers. Although polyethylene and its derivatives, namely, ultra-high molecular weight polyethylene, Vitamin-E infused polyethylene, and crosslinked polyethylene, have commonly been implemented in large joint reconstruction as a surface on which the opposing implant would articulate against, its use as a femoral and/or tibial component in total knee replacement procedures has not seen the same attention. Further, recent studies have shown that the wear debris particles from currently used polyethylene implants, e.g., tibial plates, acetabular liners, etc., activate the cell processes which induce phagocytosis via macrophages and subsequent cytokine secretion leading to bone resorption ultimately leading to osteolysis. This process is different than the bone loss seen in stress-shielding that accompanies metallic and/or ceramic based femoral implants but ultimately leads to the same results with implant loosening, bone mineral density loss, and implant failure. Additionally, the difficulty of revisionary procedures increased in the same manner as in stress-shielding if the distal femoral bone has lost bone mineral density resulting in less than ideal revisionary procedures and subsequent survival rates for the revised implant for the duration of the patient's life.

An accompanying issue related to the problem of bone mineral density loss is that of the fixation of the implant to the femoral bone. Typically, the distal femoral bone is resectioned so that the femoral knee implant will fit onto the bone. Among the technologies for ensuring fixation to the bone, two types of femoral knee implants have been used: cemented and cementless. Cemented fixation employs the use of a bone cement, typically polymethylmethacrylate (PMMA), at the bone/implant interface in an effort to reduce postoperative micromotions and migration. This type of fixation requires a strong mechanical bond between both the cement and the bone as well as the cement and the implant. While initial fixation is adequate for the cemented technique, it has been found that micromotions can occur in long-term studies. Some of the critical factors to consider when choosing this method are the potential for third-body wear, extra-articular impingement, increased surgical time, higher complexity of revision, thermal necrosis of the bone, and the potential of fat embolism during pressurization of the cement in the femoral canal. Since the bone cement commonly used is not bioabsorbale and does not degrade, its presence for the lifetime of the implant continually introduces the chance for the cement to become loose and deposit between the articulating surfaces leading to premature implant failure. Conversely, cementless fixation relies on surface topography that is conducive to attracting bone growth. This method typically relies on morphological modifications such as porosity and/or the application of biologically active coatings to the surface of the femoral knee implant in contact with the distal femoral bone. The advantages found in cementless implants are that the fixation occurs via a physiological bond as the bone grows onto the implant as well as the long term stability when compared to cemented implants. While cementless implants have shown small postoperative micromotions in the first three months, they typically reach stability after this time point and show equivalent or better survival rates compared to cemented techniques. Further, this technique does not increase the level of difficulty for revisionary procedures, should they be needed, as there is no residual cement between the bone and implant that would need to be removed in order to replace the implant. Furthermore, the wear occurring at the articulating surface of the femoral component of a knee implant occurs in everyday activities of patients (e.g., walking, squatting, etc.) and can lead to discomfort, bone damage, and ultimate implant failure and is addressed by the herein disclosed invention. Addition of wear-resistant additives to the articulating interface can increase the tribological properties of the implant while retaining a biologically favorable response and superior mechanical properties.

The bone/implant interface is typically prone to failure due to lack of a physiological bond between the implant and the native bone. The present invention aims to improve this implant adherence to the native bone via two platforms: (1) the introduction of a layer of bioadditives at the bone/implant interface and/or (2) a porous topography at the bone/implant interface containing micropores and/or macropores in an organized or unorganized manner. Both of these techniques may be used independently or together as a system and are capable of initiating and/or sustaining bone ingrowth at the bone/implant interface. Also, the presence of a physiologically-driven bond at the bone/implant interface may serve to offer improved load transfer across the interface and into the femoral bone compared to other currently used techniques such as cemented and/or cementless femoral implants. The occurrence of a physiological bond at the bone/implant interface as opposed to a non-physiological bond may lead to more efficient and relevant load-transfer across the interface therefore leading to the ability of the bone to retain its bone mineral density in accordance to Wolff's law. Additionally, the present invention has the potential to promote faster post-operative healing as the presence of bioadditives and/or porous surface found in the polymeric matrix at the bone/implant interface may elicit rapid bone ingrowth. The articulating surface of the implant comprising a polymeric based material in its virgin form or containing additives capable of increasing wear resistance at the articulating interface.

Critical factors in the selection of this technique include the necessity for good bone quality, accuracy of the bone resectioning prior to implantation, and the issue of early-stage post-operative micromotions. Furthermore, the increased price per implant, which is commonly seen as a negative for this technique, is diminished when the operative time, cement, and mixing equipment necessary for cemented techniques is taken into account. While this cementless technique is more commonly used for tibial implants, its known benefits may offer enhanced fixation, load transfer, and biointegration in a femoral knee implant.

Recent efforts to improve the fixation and load transfer between the implant and femoral bone have led to investigations into the implementation of a porous structure at the bone/implant interface. This porosity at the bone/implant interface allows for bone ingrowth into the implant therefore enhancing the overall stability and potential survival rate of the implant. Porous titanium has been a lead material choice for this particular application as its positive response to bone in-growth has been widely reported. However, even though bone in-growth in these types of porous Ti implants may be sufficient, the problem of stress-shielding still exists and may results in the same detrimental results of the presently used metallic and/or ceramic based femoral knee implants.

The present invention is directed to increasing the wear-resistance of a knee implant component as well as initiating a physiological bond at the interface between the knee implant and the native ends of the distal femoral and/or tibial bone. Addition of wear-resistant additives to the articulating interface can increase the tribological properties of the implant while retaining a biologically favorable response and superior mechanical properties.

Further, the present invention is also directed to a knee implant made via additive manufacturing.

Further, the present invention is also directed to a porous knee implant that provides improved biological response and improved fixation as compared to nonporous knee implants.

Furthermore, the present invention is also directed to a porous knee implant having a polymeric material comprising a PAEK species (PEEK, PEKK, PEKEKK, etc.).

The wear occurring at the articulating interface of orthopaedic implants occurs in everyday activates of patients post-operatively, and is addressed in the herein disclosed invention. Implementation of a low-wear polymeric species as well as the introduction of wear-resistant additives to this same polymeric species matrix can offer reduced wear-rates and subsequently longer service-lives and patient well-being. Reduction of the wear-rate by implementation of a low-wear polymeric species and/or compositing the same polymeric species with fibrous, particulate, and/or a combination of them therein, at the articulation surface will also reduce the potential for the production of wear-debris and subsequent negative biological response. Further, the wear-reinforcements can be adjusted so as to simultaneously modify the implant mechanical properties to match more closely with that of the native bone in an effort to reduce stress-shielding and therefore retain bone mineral density in the bone near the implant.

The bone/implant interface is typically prone to failure due to lack of a physiological bond between the implant and the native bone. The present invention aims to improve this implant adherence to the native bone via two platforms: (1) the introduction of a layer of bioadditives at the bone/implant interface and/or (2) a porous topography at the bone/implant interface containing micropores and/or macropores in an organized or unorganized manner. Both of these techniques may be used independently or together as a system and are capable of initiating and/or sustaining bone ingrowth at the bone/implant interface. Also, the presence of a physiologically-driven bond at the bone/implant interface may serve to offer improved load transfer across the interface and into the femoral and/or tibial bone compared to other currently used techniques such as cemented and/or cementless femoral and/or tibial implants. The occurrence of a physiological bond at the bone/implant interface as opposed to a non-physiological bond may lead to more efficient and relevant load-transfer across the interface therefore leading to the ability of the bone to retain its bone mineral density in accordance to Wolff's law. Additionally, the present invention has the potential to promote faster post-operative healing as the presence of bioadditives and/or porous surface found in the polymeric matrix at the bone/implant interface may elicit rapid bone ingrowth.

### Brief Description of the Drawings

Some exemplary embodiments of the disclosed invention are illustrated as examples but are not to be understood as limitations of the invention wherein:
FIG. 1 depicts a typical femoral knee implant showing the articulating surface **1** and the bone/implant interface surface **2.**
FIG. 2 depicts a typical femoral knee implant showing the articulating face **1,** the bone/implant interface surface **2,** and pegs **3** which are commonly implemented to aid in primary implant fixation.
FIG. 3 depicts one platform where a porous bone/implant interface surface **4** is implemented to a controlled thickness **5** from the bone/implant interface surface.
FIG. 4 depicts one platform where a porous bone/implant interface surface containing bioactive additives **6** is implemented to a controlled thickness **7** from the bone/implant interface surface.
FIG. 5 depicts one platform where a wear-resistant composite articulating surface **8** is implemented to a controlled thickness **9** from the articulating surface.
FIG. 6 depicts one platform where a wear-resistant composite articulating surface **8** is implemented to a controlled thickness **9** from the articulating surface as well as a porous bone/implant interface surface **4** is implemented to a controlled thickness **5** from the bone/implant interface surface.
FIG. 7 depicts one platform where a wear-resistant composite articulating surface **8** is implemented to a controlled thickness **9** from the articulating surface as well as a porous bone/implant interface surface containing bioactive additives **6** is implemented to a controlled thickness **7** from the bone/implant interface surface.
FIG. 8 depicts a typical tibial baseplate implant showing the tibial tray platform **10,** stabilizing fins **11,** and stabilization post **12.**
FIG. 9 depicts a typical tibial baseplate implant showing the tibial tray platform **10** as well as one platform of the herein disclosed invention comprising surface porous stabilizing fins **13** and a surface porous stabilizing post **14.**
FIG. 10 depicts a typical tibial baseplate implant showing one platform of the herein disclosed invention comprising surface porous stabilizing fins **13,** a surface porous stabilizing post **14,** and the lower side of the tibial tray platform comprising surface porosity **15.**
FIG. 11 depicts a typical tibial baseplate implant showing the tibial tray platform **10** as well as one platform of the herein disclosed invention comprising surface porous stabilizing fins containing bioactive additives **16** and a surface porous stabilizing post containing bioactive additives **17.**
FIG. 12 depicts a typical tibial baseplate implant showing one platform of the herein disclosed invention comprising surface porous stabilizing fins containing bioactive additives **16,** a surface porous stabilizing post containing bioactive additives **17,** and the lower side of the tibial tray platform comprising surface porosity containing bioactive additives **18.**
FIG. 13 depicts a typical tibial baseplate implant showing the stabilizing fins **11,** and stabilization post **12,** and a wear-reinforced composite tibial tray platform **19.**
FIG. 14 depicts a typical tibial baseplate implant showing the wear-reinforced composite tibial tray platform **19** as well as one platform of the herein disclosed invention comprising surface porous stabilizing fins **13** and a surface porous stabilizing post **14.**
FIG. 15 depicts a typical tibial baseplate implant showing the wear-reinforced composite tibial tray platform **19** as well as one platform of the herein disclosed invention comprising surface porous stabilizing fins **13,** a surface porous stabilizing post **14,** and the lower side of the tibial tray platform comprising surface porosity **15.**
FIG. 16 depicts a typical tibial baseplate implant showing the wear-reinforced composite tibial tray platform **19** as well as one platform of the herein disclosed invention comprising surface porous stabilizing fins containing bioactive additives **16** and a surface porous stabilizing post containing bioactive additives **17.**
FIG. 17 depicts a typical tibial baseplate implant showing the wear-reinforced composite tibial tray platform **19** as well as one platform of the herein disclosed invention comprising surface porous stabilizing fins containing bioactive additives **16,** a surface porous stabilizing post containing bioactive additives **17,** and the lower side of the tibial tray platform comprising surface porosity containing bioactive additives **18.**
FIG. 18 depicts the stabilization insert that holds the tibial baseplate in place within the tibial bone showing the connection to the tibial baseplate **20** and the typical bone/implant interface surface **21.**
FIG. 19 depicts the stabilization insert that holds the tibial baseplate in place within the tibial bone showing the connection to the tibial baseplate **20** and a surface porous bone/implant interface surface **22.**
FIG. 20 depicts the stabilization insert that holds the tibial baseplate in place within the tibial bone showing the connection to the tibial baseplate **20** and a surface porous bone/implant interface surface containing bioactive additives **23.**
FIG. 21 A, B and C depicts compositional data of the polymer materials containing a bioactive additive. Thermogravimetric analysis shows that the bioactive additive comprises about 20% of the blended material by weight. The polymeric material shown is poly ether ether ketone while the bioactive additive is biphasic calcium phosphate (BCP) - a calcium phosphate derivative.
FIG. 22 depicts the average pore sizes of the test specimens showing that small pores are within a range of about 0.35 mm to about 0.5 mm while large pores are defined as having an average size of about 0.55 mm to about 2 mm. Samples containing a gradient porosity are therefore a mix of small and large pores in a fashion with one layer of porosity existing below a second, dissimilar layer of porosity in a series.
FIG. 23 depicts the improvement in fixation found in a static loading pull-off force analysis when a porous surface is used at the bone-implant interface.
FIG. 24 depicts the increase in flexural response until failure when imparting a porous structure to the bone implant interface of the implant when tested under static compressive loads.

### Detailed Description of the Invention

The terminology used herein is chosen for the purpose of describing particular embodiments of the invention but are not intended to impart limitations of the invention. Herein, the term "and/or" includes any and all combinations of one or more of the associated listed items. Further, the terms "comprises" and/or "comprising" is to be understood in the specification to specify the presence of stated features, procedures, components, elements, geometries, and/or other characteristics, but do not impede the presence and/or addition of one or more further features, procedures, components, elements, geometries, and/or other characteristics thereof.

Unless otherwise defined, all terms, including those scientific and/or technical in nature, are to be understood to have the same meanings as those commonly understood by one having ordinary skill in the art of the invention. Further, terms such as those of a common nature, should be interpreted as having a meaning that is consistent with their meaning in the scope of the relevant art and/or the present disclosure and shall not be interpreted in an overly formal sense without the term being expressly defined so herein.

In describing the herein disclosed invention, it will be understood that a number of techniques and/or steps are disclosed. Each of these stated techniques and/or steps has individual benefit and each can also be used in conjunction with one or more of the other herein disclosed techniques and/or steps. Therefore, this description will refrain from repeating all possible combinations of these individual steps in an unnecessary fashion for the sake of clarity but is not to be understood as limiting the possible techniques and/or steps that lie within the scope of the invention and claims.

A new device, preferred (but not limiting) embodiments, composite specifications, and methods for implementing the present invention are discussed herein. In the following description, for purposes of explanation, specific details are set forth in order to provide a thorough understanding of the present invention. It will be evident, however, to one skilled in the art that the present invention may be practiced without these specific details.

The disclosure herein is to be considered only as an exemplification of the invention and is not to be understood as a limitation of the invention to the specific embodiments which are illustrated by the descriptions and figures herein.

The present invention will now be described by referencing the appended figures representing preferred embodiments, but not limiting potential embodiments of the present invention.

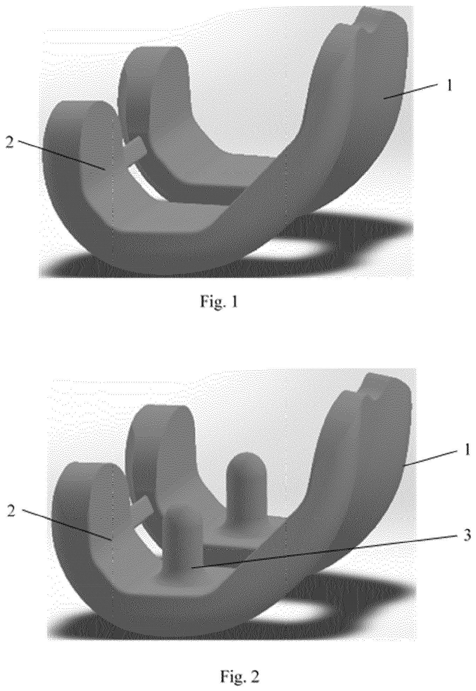

FIG. 1 depicts a typical femoral knee implant depicting the articulating surface **1** and the bone/implant interface surface **2.** FIG. 2 shows a second potential embodiment of a typical femoral implant with the articulating surface **1** and bone/implant interface **2** shown as well as fixation pegs **3** commonly used to aid in primary fixation of the implant. The articulation surface 1 in these embodiments typically undergoes the highest wear-rate in the implant as it articulates against an opposing tibial component during everyday activities. The bone/implant interface **2** generally is fixed to the distal femoral bone with the use of fixation pegs **3** and/or bone cement, typically PMMA. This fixation has adequate primary fixation, but it known to fail with long-term studies of the implants.

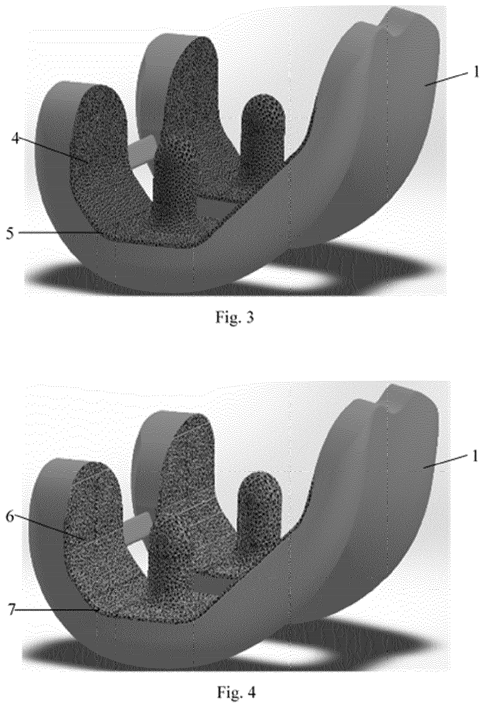

FIG. 3 depicts one possible embodiment of the present invention depicting the articulating surface **1** as well as a porous bone/implant interface **4** with a controlled thickness of porosity **5** measured from the bone/implant interface. The introduction of the porous bone/implant interface **4** may be capable of initiating more rapid bone ingrowth and subsequent long-term fixation without the use of pegs or bone cement thus removing potential stress-concentration zones and potential third-body wear particles, respectively. Further, the controlled thickness of porosity **5** allows for enhancing the physiological response from the native bone without affecting the articulating surface **1** and thus wear properties.

FIG. 4 further depicts a porous bone/implant interface containing bioactive additives **6** with a controlled thickness of porosity and bioactive additive penetration **7** measured from the bone/implant interface. The introduction of the porous bone/implant interface containing bioactive additives **6** may be capable of initiating more rapid bone ingrowth and subsequent long-term fixation without the use of pegs or bone cement thus removing potential stress-concentration zones and potential third-body wear particles, respectively. Further, the controlled thickness of porosity and bioactive additive penetration **7** allows for enhancing the physiological response from the native bone without affecting the articulating surface **1** and thus wear properties.

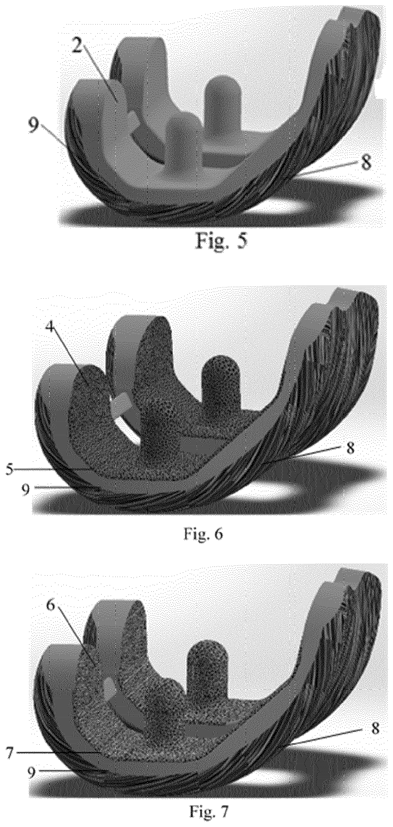

FIG. 5 depicts a modified typical femoral knee implant. In this particular embodiment, the bone/implant interface **2** remains as is found in typical femoral knee implants. The articulating surface, however, contains wear-resistant additives **8** to a controlled depth **9** as measured from the articulating surface. The presence of these wear-resistant additive may improve the wear-rate of the femoral knee implant articulating interface and lead to longer service life of the implant.

FIG. 6 depicts a femoral knee implant comprising a porous bone/implant interface **4** with a controlled depth of porosity **5** as well as a wear-resistant additive reinforced articulating surface **8** with a controlled depth of wear-resistant additive **9.** This particular embodiment may aid in initial and long-term fixation of the implant due to the porous bone/implant interface **4** as well as increase the service-life of the implant due to the wear-resistant additives **8** at the articulating surface.

FIG. 7 depicts a further potential embodiment of the present invention comprising a porous bone/implant interface containing bioactive additives **6** with a controlled thickness of porosity and bioactive additive penetration **7** measured from the bone/implant interface. The introduction of the porous bone/implant interface containing bioactive additives **6** may be capable of initiating more rapid bone ingrowth and subsequent long-term fixation without the use of pegs or bone cement thus removing potential stress-concentration zones and potential third-body wear particles, respectively. Further, the addition of wear-resistant additives **8** at the articulating surface to a controlled depth **9** as measured from the articulating interface may lead to a more resistant wear-composite at the articulating surface. This particular embodiment may lead to superior bone response and wear-resistance and therefore give greatly enhanced implant survival rates compared to currently used technologies.

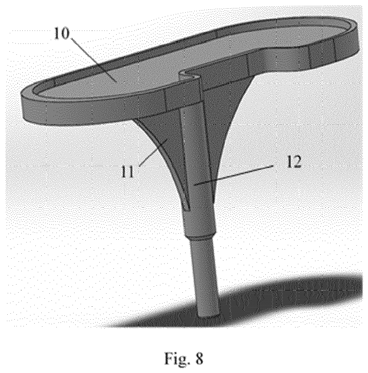

FIG. 8 depicts a typical tibial insert used in total knee replacement comprising the tibial baseplate **10,** stabilizing fins **11,** and a stabilizing post **12.** The tibial plate **10** commonly serves to hold in place the material which the articulating surface of the femoral component articulates against.

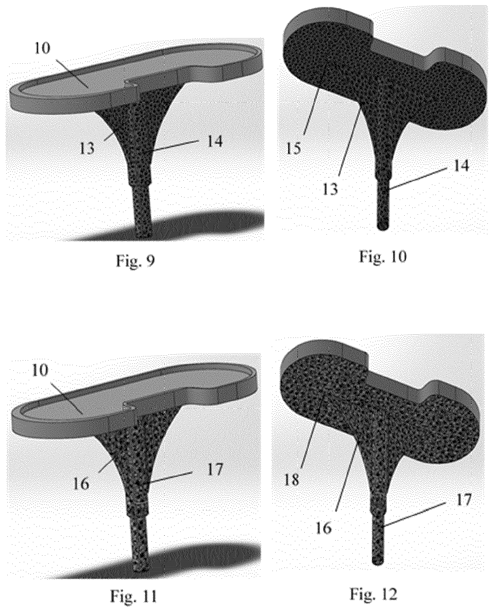

FIG. 9 depicts another potential embodiment of the present application comprising a stabilizing fins with surface porosity **13** and a stabilizing post with surface porosity **14.** Further, FIG. 10 indicates the bone/implant interface of the tibial plate with implemented surface porosity **15.** The surface porosity found on the stabilizing fins **13,** the stabilizing post **14,** and the lower side of the tibial plate **15** may initiate more efficient bone response when placed against the tibial bone upon implantation resulting in development of a stronger physiological bond and subsequent increased survival of the implant.

FIG. 11 depicts another potential embodiment of the present application comprising a stabilizing fins with surface porosity containing bioactive additives **16** and a stabilizing post with surface porosity containing bioactive additives **17.** Further, FIG. 12 indicates the bone/implant interface of the tibial plate with implemented surface porosity containing bioactive additives **18.** The surface porosity and bioactive additives found on the stabilizing fins **16,** the stabilizing post **17,** and the lower side of the tibial plate **18** may initiate more efficient bone response when placed against the tibial bone upon implantation resulting in development of a stronger physiological bond and subsequent increased survival of the implant.

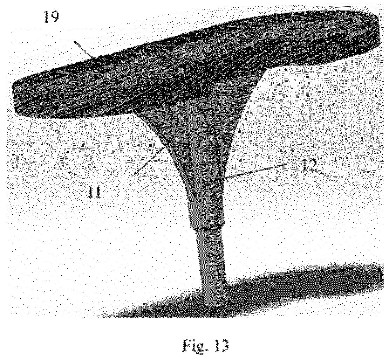

FIG. 13 depicts a typical tibial insert used in total knee replacement comprising stabilizing fins **11,** and a stabilizing post **12.** Further, the tibial baseplate containing wear-resistant additives **19** is introduced. Introduction of a tibial baseplate containing wear-resistant additives may promote greater wear-resistance to the tibial baseplate and lead to enhanced longevity of the implant.

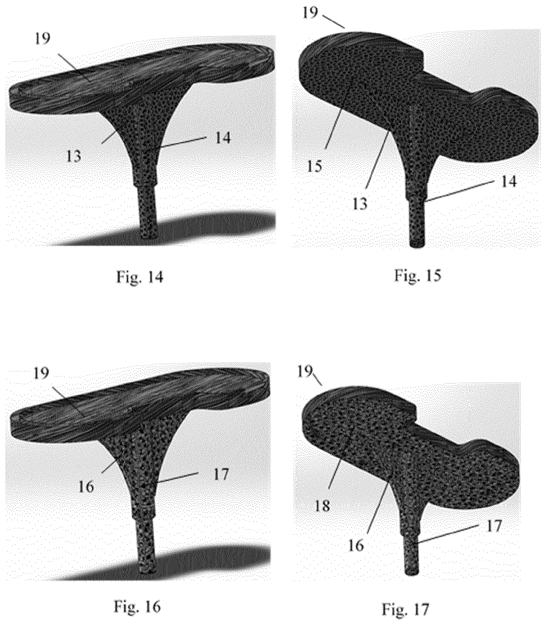

FIG. 14 depicts another potential embodiment of the present application comprising a stabilizing fins with surface porosity **13,** a stabilizing post with surface porosity **14,** and a tibial baseplate containing wear-resistant additives. Further, FIG. 15 indicates the bone/implant interface of the tibial plate containing wear-resistant additives **19** with implemented surface porosity **15.** The surface porosity found on the stabilizing fins **13,** the stabilizing post **14,** and the lower side of the tibial plate **15** may initiate more efficient bone response when placed against the tibial bone upon implantation resulting in development of a stronger physiological bond and subsequent increased survival of the implant.

FIG. 16 depicts another potential embodiment of the present application comprising a stabilizing fins with surface porosity containing bioactive additives **16,** a stabilizing post with surface porosity containing bioactive additives **17,** and a tibial baseplate containing wear-resistant additives. Further, FIG. 17 indicates the bone/implant interface of the tibial plate containing wear-resistant additives **19** with implemented surface porosity containing bioactive additives **18.** The surface porosity and bioactive additives found on the stabilizing fins **16,** the stabilizing post **17,** and the lower side of the tibial plate **18** may initiate more efficient bone response when placed against the tibial bone upon implantation resulting in development of a stronger physiological bond and subsequent increased survival of the implant.

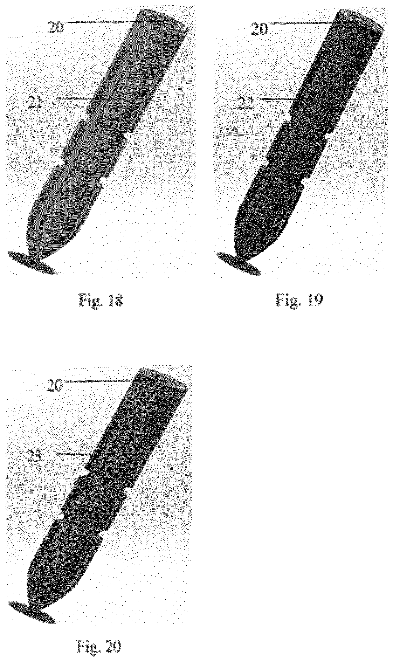

FIG. 18 depicts the tibial insert typically used to further stabilize the tibial baseplate with the connection to the tibial baseplate **27** the bone/implant interface surface **28.**

FIG. 19 depicts a potential embodiment of the invention comprising the connection to the tibial baseplate **27** and a porous surface at the bone/implant interface **29.** The surface porosity found on the bone/implant interface of the tibial insert **29** may initiate more efficient bone response when placed against the tibial bone upon implantation resulting in development of a stronger physiological bond and subsequent increased survival of the implant.

FIG. 20 depicts a potential embodiment of the invention comprising the connection to the tibial baseplate **27** and a porous surface containing bioactive additives at the bone/implant interface **30.** The surface porosity and bioactive additives found on the bone/implant interface of the tibial insert **30** may initiate more efficient bone response when placed against the tibial bone upon implantation resulting in development of a stronger physiological bond and subsequent increased survival of the implant.

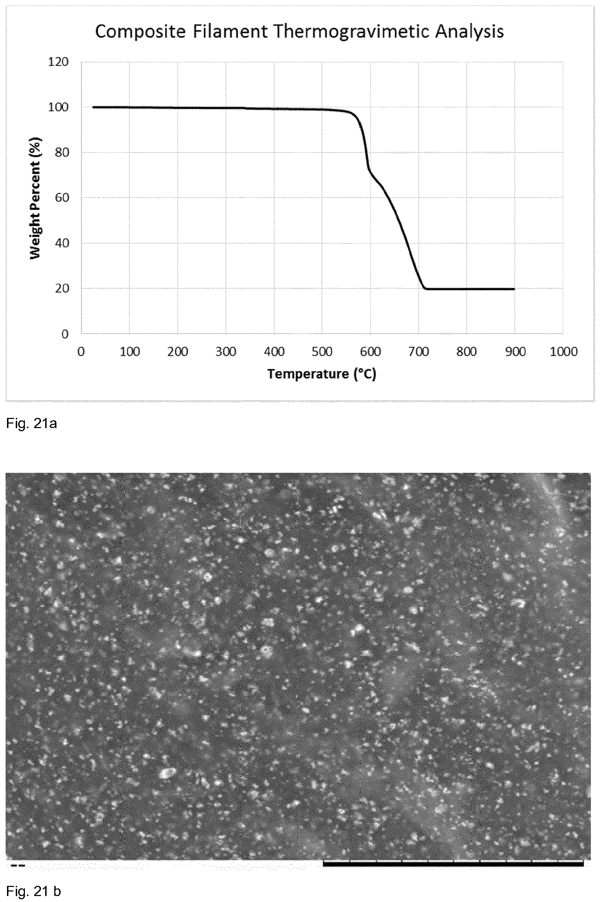

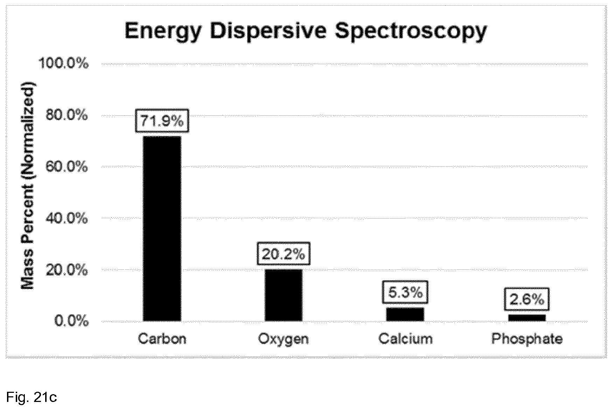

FIG. 21 depicts compositional data of the polymer materials containing a bioactive additive. Thermogravimetric analysis (FIG. 21A) shows that the bioactive additive comprises about 20% of the blended material by weight. The polymeric material shown is poly ether ether ketone while the bioactive additive is biphasic calcium phosphate (BCP) - a calcium phosphate derivative. Relative amounts of the chemical constituents carbon, oxygen, calcium, and phosphate are also shown as a result of energy dispersive spectroscopy (Fig. 21B & FIG. 21C) to verify chemical composition and presence of bioactive additive BCP.

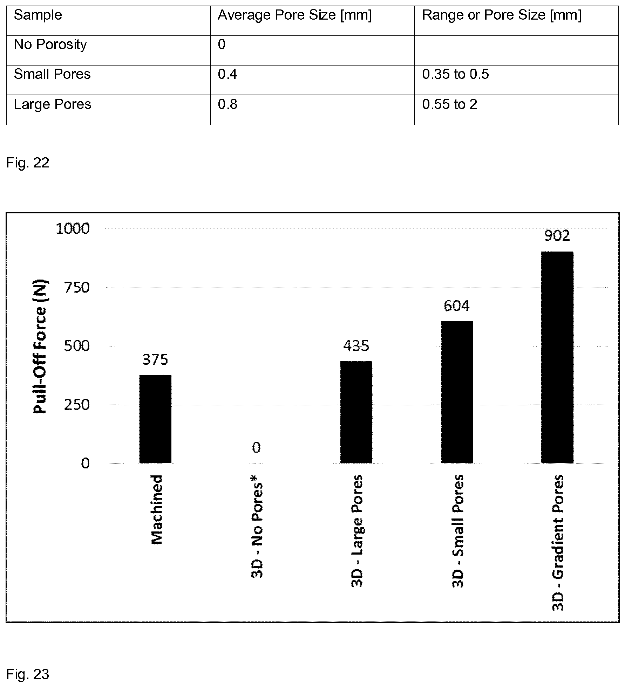

FIG. 22 depicts the average pore sizes of the test specimens showing that small pores are within a range of about 0.350mm to about 0.500mm while large pores are defined as having an average size of about 0.550mm to about 2mm. Samples containing a gradient porosity are therefore a mix of small and large pores in a fashion with one layer of porosity existing below a second, dissimilar layer of porosity.

FIG. 23 depicts the improvement in fixation found in a static loading pull-off force analysis when a porous surface is used at the bone-implant interface.

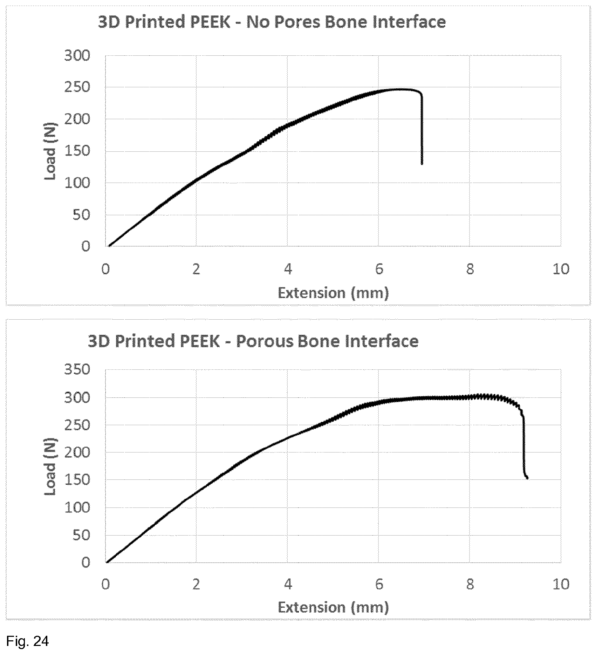

FIG. 24 depicts the increase in flexural response until failure when imparting a porous structure to the bone implant interface of the implant when tested under static compressive loads.

A polymeric-based knee implant comprising an articulating surface and a bone/implant interface.

Preferably the implant further comprises fixation pegs.

The bone/implant interface is porous. The bone/implant interface has a pore size preferably of 0.1 mm to 10 mm, more preferably of 0.2 mm to 5 mm, and most preferably of 0.3 mm to 1 mm. Preferably the pore sizes are determined by electron microscopy (preferably SEM) or optical microscopy. Small pores are defined within a range of about 0.35 mm to about 0.5 mm while large pores are defined as having an average size of about 0.55 mm to about 2 mm.

Preferably the bone/implant interface has gradient pores. Preferably, gradient porosity is a mix of small and large pores, preferably in a fashion with one layer of porosity existing below a second, dissimilar layer of porosity in a series.

The articulating surface comprises a high wear resistant bioinert composition.

The implant consists of one or more components and at least one component comprises a bioinert polymer. The at least one or more components comprises a bioactive additive, which is biphasic calcium phosphate.

Suitable bioinert polymers include but are not limited to PAEK species (PEEK, PEKK, PEKEKK, etc.).

The bioactive additive can further comprise a phosphate derivative, preferably apatites and calcium phosphates, more preferably the apatites comprise hydroxyapatites and fluorohydroxyapatites. The phosphates comprise preferably alpha and/or beta tri calcium phosphate. A bioactive additive can also include an osteoconductive additive. An osteoconductive additive is an additive that can promote bone growth.

Suitably, the bioactive material is a material which elicits a specific biological response at the interface of the material, which results in a formation of a bond between tissue and polymeric material. Preferably the one or more components of the implant comprises a functionally graded layer of bioactive additive.

Preferably the one or more components of the implant comprises a bioinert polymer, a bioactive additive, an osteoconductive additive, or any combination or mixture thereof. Preferably the one or more components of the implant comprises a bioinert polymer, wherein the bone/implant interface is porous. Preferably the one or more components of the implant comprises a bioinert polymer, wherein the articulating surface comprises wear-resistant additives.

Preferably the one or more components of the implant comprises a functionally graded layer of wear-resistant additive.

Preferably the articulating surface comprises a functionally graded layer of wear-resistant additive. Preferably the wear-resistant additive is carbon, glass, polymeric, ceramic, metallic, or any combination or mixture thereof.

Preferably the one or more components of the implant comprises a bioinert polymer, wherein the articulating surface comprises wear-resistant additives, and wherein the bone/implant interface comprises a bioactive additive, an osteoconductive additive, or any combination or mixture thereof. Preferably the one or more components of the implant comprises a bioinert polymer, wherein the articulating surface comprises wear-resistant additives, and wherein the bone/implant interface is porous. Preferably the one or more components of the implant comprises a bioinert polymer and a bioactive additive, wherein the articulating surface comprises wear-resistant additives, and wherein the bone/implant interface is porous.

Preferably the implant comprises a reinforcing additive. Preferably the reinforcing additive is a fibrous material, particulate material, or any combination or mixture thereof.

Preferably the fibrous material is carbon-based. Preferably the fibrous material is carbon fibers, pitch-derived carbon fibers, pan-derived carbon fibers, or any combination or mixture thereof.

Preferably the fibrous material is polymeric, glass, ceramic, or any combination or mixture thereof. Preferably the particulate material is polymeric, metallic, ceramic, or any combination or mixture thereof.

Preferably the implant is fabricated through blending, compounding, extruding, injection molding, multi-component injection molding, co-injection molding, two-shot injection molding, injection-compression molding, compression molding, hot pressing, hot isotactic pressing, additive manufacturing, or a combination thereof.

Preferably the porosity of the porous knee implant is fabricated by abrasive blasting, salt-leaching, matrix dissolution, additive manufacturing, or a combination thereof, more preferably by additive manufacturing.

Preferably the implant is fabricated by additive manufacturing after blending a bioinert polymer with a bioactive additive to form a blended material.

Preferably said blended material is achieved through twin-screw melt compounding into a filament.

Preferably the filament has a diameter between 1.5 mm and 3.25 mm. Preferably the filament contains up to about 20 wt% of said bioactive additive.

Preferably the polymeric-based knee implant is printed via additive manufacturing with a print head temperature of between 380°C and 440°C. Preferably the implant is printed via additive manufacturing with a print bed temperature of between 110°C and 160°C. Preferably the implant is printed via additive manufacturing with a print speed of between 10 mm/sec and 40 mm/sec.

Preferably the implant is thermally annealed, preferably the annealing is carried out at a temperature between 140°C and 250°C.

In preferred embodiments, the polymeric material for the device would be one from polyaryletherketone (PAEK polymers, including but not limited to PEEK, PEKK, PEKEKK, and other polymerization species of the parent species of PAEKs consisting of a backbone of alternating ether and ketone bonds contacting, or not containing, crosslinked polymer chains), polyetheretherketone (PEEK, poly-ether-ether-ketone, and other embodiments of the parent polymer, PAEKs, containing, or not containing, crosslinked polymeric chains).

In preferred embodiments, the polymeric species, including but not limited to polyaryletherketone (PAEK polymers, including but not limited to PEEK, PEKK, PEKEKK, and other polymerization species of the parent species of PAEKs consisting of a backbone of alternating ether and ketone bonds contacting, or not containing, crosslinked polymer chains), polyetheretherketone (PEEK, poly-ether-ether-ketone, and other embodiments of the parent polymer, PAEKs, containing, or not containing, crosslinked polymeric chains), would exist as the main species in the device. Reinforcing fibers, including but not limited to carbon fibers (both pitch-based and PAN-based), glass fibers, polymeric fibers, and/or ceramic fibers may exist within the polymeric matrix of the device as a methods of reinforcement and enhancement biological response.

In preferred embodiments, the polymeric species, including but not limited to polyaryletherketone (PAEK polymers, including but not limited to PEEK, PEKK, PEKEKK, and other polymerization species of the parent species of PAEKs consisting of a backbone of alternating ether and ketone bonds contacting, or not containing, crosslinked polymer chains), polyetheretherketone (PEEK, poly-ether-ether-ketone, and other embodiments of the parent polymer, PAEKs, containing, or not containing, crosslinked polymeric chains), would exist as the main component in the device. Reinforcing particulates, including but not limited to carbon particles (both pitch-based and PAN-based), glass particulates, metal particulates, ceramic particulates, and/or any combination therein may exist within the polymeric matrix of the device as a methods of enhancement of mechanical and wear properties as well as enhancing biological response.

Preferably the polymeric species does not contain any polaethylene (PE, UHMWPE, crosslinked PE, Vitamin-E infused PE, and other embodiments and compounds of the parent polymer).

In preferred embodiments, the device is one that has been manufactured via thermal method. These methods include, but are not limited to, compression molding, injection molding, additive manufacturing, or any combination therein. As an example, but not a fabrication limitation, the polymeric species, either neat/virgin or containing additives, may be forcibly injected into a design mold of the specific application geometry. Conversely, the polymeric species, either neat/virgin or containing additives, may be extruded or otherwise mixed thermally and forced through a die and the subsequent material melt placed into a mold and formed to shape.

The following examples are put forth so as to provide those of ordinary skill in the art with a complete disclosure and description of how the compositions, articles, devices and/or methods claimed herein are made and evaluated, and are intended to be purely exemplary of the invention and are not intended to limit the scope of what the inventors regard as their invention. Efforts have been made to ensure accuracy with respect to numbers (e.g., amounts, temperature, etc.), but some errors and deviations should be accounted for. Unless indicated otherwise, parts are parts by weight, temperature is in ° C. or is at ambient temperature, and pressure is at or near atmospheric.

Example 1: The bioinert polymer (PEEK) was blended with a bioactive added calcium phosphate derivative known as biphasic calcium phosphate (BCP) through a dry blending procedure followed by thermal processing via twin-screw compounding. First, the respective materials were weighed out in powder form to comprise a total compositional ratio of 80 wt% polymer (80g PEEK) and 20 wt% biphasic calcium phosphate (20g BCP) using an inversion blender. The blended powders were then dried overnight at 80°C. After drying the powder blend was processed in a twin-screw compounder with temperature profiles between 250°C and 410°C. The resultant extrudate was then drawn into a composite filament and collected for use in fused filament fabrication.

Example 2: Fused filament fabrication of the composite filament of EXAMPLE 1 was carried out as follows. The PEEK + 20% BCP constructs were printed using the 3NTR A4v3 fused filament fabrication printer. The constructs were printed using a nozzle temperature of 410 °C, a bed temperature of 135 °C, and a chamber temperature of 75 °C. The first layer of the construct was printed at a thickness of 0.4 mm while the rest of the part was printed at a layer height of 0.1 mm. A raft was used for each print to prevent the parts from warping and to better adhere to the build plate. This procedure was carried out to fabricate all testing samples with the variation existing only in the geometries of pore sizes as selected.

Example 3: As depicted in Fig 23, fabricated samples of the blended composite material from EXAMPLES 1 and 2 were exposed to static loading in order to measure pull-off force after fixation to synthetic bone with poly(methyl methacrylate) (PMMA) bone cement until a pull-off force was achieved that caused the sample to separate from the PMMA/synthetic bone construct. The PMMA used was Stryker Simplex P at 20°C with mixing done in a vacuum and the cement being added after approximately 5 minutes. Impaction of the testing construct was carried out using a surgical hammer and manual pressure to seat the implant. After 1 hour of setting time, the implant construct was affixed to a hydraulic actuator through a ½" stud on a MTS Mini Bionix tensile tester. The synthetic bone construct was affixed to the lower side of the test area using a vice grip. Tests were carried out at a constant displacement rate of 0.008 mm/s and a data sampling rate of 100 Hz until the load reached 10% of peak load which was deemed to be after failure. The data demonstrates that the samples that contained small pores and gradient pores had a higher pull off force, thus a better anchoring effect than the samples with no pores and large pores. The sample with the best anchoring effect was the sample with the gradient pores.

Example 4: As depicted in Fig 24, the no pores bone interface and porous bone interface constructs were printed as described in the above examples and exposed to static loading on the proximal end of the implant after affixing the distal end into an epoxy resin in order to measure the load required for failure of the implant in a bending environment. The implants were potted in a fixture filled with two part epoxy resin with an embedding height of 25mm measured from bottom flat surface of the implant condyle to the top of the resin potting fixture. The potted implants were placed inside an oven at 40°C for one hour, taken out of the oven and let to rest for an additional hour at room temperature to allow the resin to fully cure. The specimens were then tested using an Instron 3366B dual column frame in compression. Each implant was placed in between two compression platens with the top platen allowed to slide along the top surface of the implant's condyle. The compression test was then conducted until failure. The porous bone interface showed that it held more load, and showed greater extension before it failed than the no pore bone interface.

Example 5: As depicted in Fig. 21a, thermogravimetric analysis and energy dispersive spectroscopy (EDAX) was carried out on the composite filament to quantify weight percentage of the bioactive additive, biphasic calcium phosphate, in the composite material. Approximately 25mg of sample material was placed into a TGA Q500 under an air atmosphere and exposed to a temperature profile as follows: equilibration at 25°C for at least 5 minutes followed by a temperature ramp at 10°C/min up to 900°C and then held isothermally for 5 minutes. The reported remaining mass was taken as the amount of bioactive additive within the composite material. EDAX results were gathered by use of a Hitachi TM3030 table-top SEM with an accelerating voltage of 15kV. Relative amounts of chemical constituents of carbon, oxygen, calcium, and phosphate also were used to verify amount of biphasic calcium phosphate (Fig. 21c).

## Claims

1. A polymeric-based knee implant comprising an articulating surface and a bone/implant interface; wherein the bone/implant interface is porous; and
wherein the implant has a polymeric material consisting of a PAEK species, preferably PEEK, PEKK, PEKEKK,
wherein the bone/implant interface comprises bioactive additives,
wherein the bioactive additive is a calcium phosphate derivative,
wherein the calcium phosphate derivative is biphasic calcium phosphate.

2. The polymeric-based knee implant of claim 1, wherein the porous bone/implant interface has a pore size of preferably 0.1 mm to 10 mm, more preferably 0.2 mm to 5 mm, and most preferably 0.3 mm to 1 mm.

3. The polymeric-based knee implant of claim 1, wherein the implant further comprises fixation pegs.

4. The polymeric-based knee implant of claim 1, consisting of one or more components, wherein at least one component of the implant comprises a bioinert polymer.

5. The polymeric-based knee implant of claim 4, wherein the one or more components comprises a bioinert polymer, wherein the articulating surface comprises wear-resistant additives.

6. The polymeric-based knee implant of claim 5, wherein the wear-resistant additive is carbon, glass, polymeric, ceramic, metallic, or any combination or mixture thereof.

7. The polymeric-based knee implant of claim 1, wherein the one or more components of the implant comprises a bioinert polymer and a bioactive additive, wherein the articulating surface comprises wear-resistant additives, and wherein the bone/implant interface is porous.

8. The polymeric-based knee implant as in any preceding claim, wherein the implant is fabricated through blending, compounding, extruding, injection molding, multi-component injection molding, co-injection molding, two-shot injection molding, injection-compression molding, compression molding, hot pressing, hot isotactic pressing, additive manufacturing, or a combination thereof.

9. The polymeric-based knee implant of claim 1, wherein the porosity is fabricated by abrasive blasting, salt-leaching, matrix dissolution, additive manufacturing, or a combination thereof, preferably by additive manufacturing.

10. The polymeric-based knee implant of claim 9, wherein the implant is fabricated by additive manufacturing after blending a bioinert polymer with a bioactive additive to form a blended material.

11. The polymeric-based knee implant of claim 10, wherein the fabrication of the blended material is achieved through twin-screw melt compounding into a filament.

12. The polymeric-based knee implant of claim 11, wherein the filament has a diameter between 1.5mm and 3.25mm.

13. The polymeric-based knee implant of claim 11, wherein said filament contains up to about 20wt% of the bioactive additive.

14. The polymeric-based knee implant of claim 9, wherein the implant is printed via additive manufacturing with a print head temperature of between 380°C and 440°C.

15. The polymeric-based knee implant of claim 9, wherein the implant is printed via additive manufacturing with a print speed of between 10mm/sec and 40mm/sec.

16. The polymeric-based knee implant as in any preceding claim, wherein the implant is thermally annealed.

## Patentansprüche

1. Polymerbasiertes Knieimplantat, eine Gelenkoberfläche und eine Knochen/Implantat-Grenzfläche umfassend; wobei die Knochen/Implantat-Grenzfläche porös ist; und wobei das Implantat ein Polymermaterial aufweist, das aus einer PAEK-Spezies, vorzugsweise PEEK, PEKK, PEKEKK, besteht,
wobei die Knochen/Implantat-Grenzfläche bioaktive Zusatzstoffe enthält,
wobei der bioaktive Zusatzstoff ein Calciumphosphat-Derivat ist,
wobei das Calciumphosphat-Derivat bipolares Calciumphosphat ist.

2. Polymerbasiertes Knieimplantat nach Anspruch 1, wobei die poröse Knochen/Implantat-Grenzfläche eine Porengröße vorzugsweise von 0,1 mm bis 10 mm, besonders bevorzugt von 0,2 mm bis 5 mm und ganz besonders bevorzugt von 0,3 mm bis 1 mm, aufweist.

3. Polymerbasiertes Knieimplantat nach Anspruch 1, wobei das Implantat weiterhin Fixationsstifte umfasst.

4. Polymerbasiertes Knieimplantat nach Anspruch 1, aus einer oder mehreren Komponenten bestehend, wobei mindestens eine Komponente des Implantats ein bioinertes Polymer enthält.

5. Polymerbasiertes Knieimplantat nach Anspruch 4, wobei die eine oder die mehreren Komponenten ein bioinertes Polymer enthalten, wobei die Gelenkoberfläche verschleißfeste Zusatzstoffe enthält.

6. Polymerbasiertes Knieimplantat nach Anspruch 5, wobei der verschleißfeste Zusatzstoff Carbon, Glas, Polymer, Keramik, Metall oder jede Kombination oder Mischung davon ist.

7. Polymerbasiertes Knieimplantat nach Anspruch 1, wobei die eine oder die mehreren Komponenten des Implantats ein bioinertes Polymer und einen bioaktiven Zusatzstoff enthalten, wobei die Gelenkoberfläche verschleißfeste Zusatzstoffe enthält und wobei die Knochen/Implantat-Grenzfläche porös ist.

8. Polymerbasiertes Knieimplantat nach einem der vorhergehenden Ansprüche, wobei das Implantat durch Vermengen, Vermischen, Extrudieren, Spritzgießen, Mehrkomponentenspritzgießen, Co-Spritzgießen, Zweistufenspritzgießen, Spritzprägen, Formpressen, Heißpressen, isotaktisches Heißpressen, Additivherstellung oder eine Kombination davon angefertigt wird.

9. Polymerbasiertes Knieimplantat nach Anspruch 1, wobei die Porigkeit durch Abstrahlen, Herauslösen von Salz, Matrixauflösung, Additivherstellung oder eine Kombination davon, vorzugsweise durch Additivherstellung, angefertigt wird.

10. Polymerbasiertes Knieimplantat nach Anspruch 9, wobei das Implantat durch Additivherstellung nach Vermengen eines bioinerten Polymers mit einem bioaktiven Zusatzstoff, um ein vermengtes Material auszubilden, angefertigt wird.

11. Polymerbasiertes Knieimplantat nach Anspruch 10, wobei die Anfertigung des vermengten Materials durch Doppelschneckenschmelzmischen zu einem Filament erzielt wird.

12. Polymerbasiertes Knieimplantat nach Anspruch 11, wobei das Filament einen Durchmesser zwischen 1,5 mm und 3,25 mm aufweist.

13. Polymerbasiertes Knieimplantat nach Anspruch 11, wobei das Filament bis zu ungefähr 20 Masse-% des bioaktiven Zusatzstoffs enthält.

14. Polymerbasiertes Knieimplantat nach Anspruch 9, wobei das Implantat durch Additivherstellung mit einer Druckkopftemperatur zwischen 380 °C und 440 °C gedruckt wird.

15. Polymerbasiertes Knieimplantat nach Anspruch 9, wobei das Implantat durch Additivherstellung mit einer Druckgeschwindigkeit zwischen 10 mm/s und 40 mm/s gedruckt wird.

16. Polymerbasiertes Knieimplantat nach einem der vorhergehenden Ansprüche, wobei das Implantat thermisch getempert wird.

## Revendications

1. Implant de genou à base de polymère comprenant une surface d'articulation et une interface os/implant ; dans lequel l'interface os/implant est poreuse ; et
dans lequel l'implant a un matériau polymère consistant en une espèce de PAEK, de préférence PEEK, PEKK, PEKEKK,
dans lequel l'interface os/implant comprend des additifs bioactifs,
dans lequel l'additif bioactif est un dérivé de phosphate de calcium,
dans lequel le dérivé de phosphate de calcium est un phosphate de calcium biphasique.

2. Implant de genou à base de polymère de la revendication 1, dans lequel l'interface os/implant poreuse a une taille de pores de préférence de 0,1 mm à 10 mm, mieux de 0,2 mm à 5 mm, et idéalement de 0,3 mm à 1 mm.

3. Implant de genou à base de polymère de la revendication 1, dans lequel l'implant comprend en outre des goujons de fixation.

4. Implant de genou à base de polymère de la revendication 1, consistant en un ou plusieurs composants, dans lequel au moins un composant de l'implant comprend un polymère bioinerte.

5. Implant de genou à base de polymère de la revendication 4, dans lequel le ou les composants comprennent un polymère bioinerte, dans lequel la surface d'articulation comprend des additifs résistant à l'usure.

6. Implant de genou à base de polymère de la revendication 5, dans lequel l'additif résistant à l'usure est du carbone, du verre, polymère, céramique, métallique, ou toute combinaison ou tout mélange de ceux-ci.

7. Implant de genou à base de polymère de la revendication 1, dans lequel le ou les composants de l'implant comprennent un polymère bioinerte et un additif bioactif, dans lequel la surface d'articulation comprend des additifs résistant à l'usure, et dans lequel l'interface os/implant est poreuse.

8. Implant de genou à base de polymère selon une quelconque revendication précédente, dans lequel l'implant est fabriqué par mélange, compoundage, extrusion, moulage par injection, moulage par injection multicomposant, moulage par co-injection, moulage par injection en deux passes, moulage par injection-compression, moulage par compression, pressage à chaud, pressage isostatique à chaud, fabrication additive, ou une combinaison de ceux-ci.

9. Implant de genou à base de polymère de la revendication 1, dans lequel la porosité est fabriquée par projection d'abrasifs, lixiviation au sel, dissolution de matrice, fabrication additive, ou une combinaison de ceux-ci, de préférence par fabrication additive.

10. Implant de genou à base de polymère de la revendication 9, dans lequel l'implant est fabriqué par fabrication additive après mélange d'un polymère bioinerte avec un additif bioactif pour former un matériau mélangé.

11. Implant de genou à base de polymère de la revendication 10, dans lequel la fabrication du matériau mélangé est réalisée par compoundage à l'état fondu à double vis en un filament.

12. Implant de genou à base de polymère de la revendication 11, dans lequel le filament a un diamètre compris entre 1,5 mm et 3,25 mm.

13. Implant de genou à base de polymère de la revendication 11, dans lequel ledit filament contient jusqu'à environ 20 % en poids de l'additif bioactif.

14. Implant de genou à base de polymère de la revendication 9, dans lequel l'implant est imprimé par fabrication additive avec une température de tête d'impression comprise entre 380 °C et 440 °C.

15. Implant de genou à base de polymère de la revendication 9, dans lequel l'implant est imprimé par fabrication additive avec une vitesse d'impression comprise entre 10 mm/s et 40 mm/s.

16. Implant de genou à base de polymère selon une quelconque revendication précédente, dans lequel l'implant est recuit thermiquement.
